# EUROPEAN PATENT APPLICATION

(11) **EP 4 640 660 A1**
(43) Date of publication of application: **29.10.2025**
(21) Application number: 24891717.1
(22) Date of filing: 08.11.2024
(51) Int. Cl.: C07C 29/80, C07C 29/82, C07C 29/04, C07C 31/10, C07C 31/12

(54) **METHOD FOR PURIFYING ISOPROPYL ALCOHOL**

(30) Priority: 15.11.2023 KR 20230158482; 25.10.2024 KR 20240147824
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR); LEE, Moon Yong, Daejeon 34122 (KR)
(74) Representative: Goddar, Heinz J.
(86) International application number: PCT/KR2024/017587
(87) International publication number: WO 2025/105771

(57) **Abstract**

The present invention provides a method for preparing isopropyl alcohol, the method including: cooling a reaction product and supplying the cooled reaction product to an absorption column; and supplying a lower discharge stream of the absorption column including isopropyl alcohol from the absorption column to an isopropyl alcohol purifying section including first to fourth columns, and supplying an upper discharge stream of the absorption column including propylene to a gas purifying section including fifth and sixth columns, wherein all or a portion of the reaction product is cooled by heat exchange with one or more of a lower discharge stream of the fourth column and a side discharge stream of the fifth column, an upper discharge stream of the second column is heat-exchanged with a lower discharge stream of the sixth column, and an upper discharge stream of the third column is heat-exchanged with one or more of the lower discharge stream of the first column and a lower discharge stream of the second column.

## Description

### [Technical Field]

### CROSS-REFERENCE TO RELATED APPLICATION

The present application claims the benefit of priorities to Korean Patent Application No. 10-2023-0158482, filed on November 15, 2023, and Korean Patent Application No. 10-2024-0147824, filed on October 25, 2024, the entire contents of which are incorporated herein by reference.

### Technical Field

The present invention relates to a method for purifying isopropyl alcohol, and in particular, to a method for purifying isopropyl alcohol from a reaction product of an isopropyl alcohol preparation process, which may reduce energy consumption and process costs.

### [Background Art]

Isopropyl alcohol (IPA) is recognized as an excellent solvent in a variety of industries and applications because it has an ability to dissolve a wide range of substances, rapid vaporization, and relatively low toxicity. Isopropyl alcohol is an essential substance in a variety of manufacturing industries, health care, and direct consumer applications.

In an isopropyl alcohol preparation process, for example, propylene and water are used as raw material components. In this case, the propylene and water react to produce isopropyl alcohol. The reaction product of the isopropyl alcohol preparation process includes various types of impurities and by-products such as diisopropyl ether (DIPE), acetone, n-propyl alcohol (NPA), and hexanol, in addition to isopropyl alcohol, an unreacted propylene monomer, and unreacted water.

In order to obtain isopropyl alcohol from the reaction product, a purification process of isopropyl alcohol is required. Therefore, in order to obtain high-purity isopropyl alcohol, the purification process of isopropyl alcohol needs to be highly efficient, and at the same time, an improved design is required from an economic perspective that may reduce not only energy consumption but also operating costs and facility costs.

### [Disclosure]

### [Technical Problem]

In order to solve the problems mentioned in the background art, an object of the present invention is to provide a method for purifying isopropyl alcohol that may obtain high-purity isopropyl alcohol, and at the same time, may reduce energy consumption and reduce operating costs and facility costs.

However, the problems to be solved by the present application are not limited to the object described above, and other problems not described will be clearly understood by those skilled in the art from the following description.

### [Technical Solution]

In one general aspect, a method for preparing isopropyl alcohol includes: reacting a propylene monomer and water to prepare a reaction product including propylene and isopropyl alcohol; cooling the reaction product and supplying the cooled reaction product to an absorption column; and supplying a lower discharge stream of the absorption column including isopropyl alcohol from the absorption column to an isopropyl alcohol purifying section including first to fourth columns, and supplying an upper discharge stream of the absorption column including propylene to a gas purifying section including fifth and sixth columns, wherein the isopropyl alcohol included in the lower discharge stream of the absorption column supplied to the isopropyl alcohol purifying section is obtained by sequentially passing through a lower discharge stream of the first column, a first side discharge stream of the second column, a first region lower discharge stream of the third column, and an upper discharge stream of the fourth column, the propylene included in the upper discharge stream of the absorption column supplied to the gas purifying section is obtained by sequentially passing through an upper discharge stream of the fifth column and a side discharge stream of the sixth column, all or a portion of the reaction product is cooled by heat exchange with one or more of a lower discharge stream of the fourth column and a side discharge stream of the fifth column, an upper discharge stream of the second column is heat-exchanged with a lower discharge stream of the sixth column, and an upper discharge stream of the third column is heat-exchanged with one or more of the lower discharge stream of the first column and a lower discharge stream of the second column.

### [Advantageous Effects]

According to the method for purifying isopropyl alcohol of the present invention, a feed including isopropyl alcohol, water, and various by-products as a reaction product of propylene and water is effectively purified, such that high-purity isopropyl alcohol may be finally obtained. In addition, unreacted propylene included in the reaction product may be recovered with a high purity and then may be reused in the reaction for preparing isopropyl alcohol.

As a process performed by at least two distillation columns in the isopropyl alcohol purifying section is performed in one distillation column, the reboiler duty that has been required for the operation of at least two distillation columns in the related art may be saved (energy saving), and the facility costs and operating costs of the device may be reduced by reducing the number of distillation columns.

In addition, the heat energy consumption that has been required for the operation of the distillation columns in the related art may be reduced through heat exchange between the distillation columns throughout the entire process for preparing isopropyl alcohol, including the isopropyl alcohol purifying section for recovering isopropyl alcohol after the reaction of propylene and water and the gas purifying section for recovering unreacted propylene.

### [Description of Drawings]

FIG. 1 is a flow diagram of the overall process of a method for preparing isopropyl alcohol according to an embodiment of the present invention.
FIG. 2 is a flow diagram showing a process between a reactor and an absorption column in a process of a method for preparing isopropyl alcohol according to an embodiment of the present invention.
FIG. 3 is a flow diagram showing a process for an isopropyl alcohol purifying section in a process of a method for preparing isopropyl alcohol according to an embodiment of the present invention.
FIG. 4 is a flow diagram showing a process for a gas purifying section in a process of a method for preparing isopropyl alcohol according to an embodiment of the present invention.
FIG. 5 is a process flow diagram of a method for purifying isopropyl alcohol according to a comparative example.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

With regard to the description of the drawings, similar reference numerals may be used to refer to similar or related components.

A singular form of a noun corresponding to an item may include one or more of the items, unless the relevant context clearly indicates otherwise.

In the present disclosure, each of such phrases as "A or B", "at least one of A and B", "at least one of A or B", "A, B, or C"**,** "at least one of A, B, and C"**,** and "at least one of A, B, or C" may include any one of or all possible combinations of the items enumerated together in one of the corresponding phrases.

The term "and/or" includes a combination of a plurality of related components or any one of the plurality of related components.

The terms such as "1st" and "2nd" or "first" and "second" may be used to simply distinguish a corresponding component from another component, and do not limit the corresponding components in other aspects (for example, importance or order).

In addition, the terms such as "front surface", "rear surface", "upper surface", "lower surface", "side surface", "left side", "right side", "upper portion", and "lower portion" used in the present application are defined based on the drawings, and the shape and position of each component are not limited by the terms.

The term "include" or "have" specifies the presence of features, numerals, steps, operations, components, parts described in the present disclosure, or a combination thereof, but does not preclude the presence or addition of one or more other features, numerals, steps, operations, components, parts, or a combination thereof.

When a component is said to be "connected", "coupled", "supported", or "contacted" with another component, this includes not only when components are directly connected, coupled, supported, or contacted, but also when components are indirectly connected, coupled, supported, or contacted through a third component.

When a component is said to be "on" another component, this includes not only when the component is in contact with another component, but also when there is another component between two components.

The term "stream" used in the present application may refer to a flow of a fluid in a process, and may also refer to a fluid flowing through a pipe itself. Specifically, the stream may refer to both a fluid flowing through a pipe connecting respective devices to each other itself and a flow of a fluid. In addition, the fluid may include any one or more components of gas, liquid, and solid.

The term "upper portion" used in the present application refers to a point at a height of 0% to 10% from the uppermost portion of a device downwards, unless otherwise specified, and specifically, may refer to the uppermost portion (the top). In addition, the term "lower portion" refers to a point at a height of 90% to 100% from the uppermost portion of the device downwards, and specifically, may refer to the lowest portion (the bottom).

In addition, the term "pressure" as referred to in the present application refers to a gauge pressure measured under atmospheric pressure conditions.

Meanwhile, unless otherwise specified herein, an operating pressure of a column refers to a pressure at an upper portion of the column, and an operating temperature of the column refers to a temperature at a lower portion of the column.

An embodiment of the present invention relates to a method for purifying isopropyl alcohol (IPA). Hereinafter, the method for purifying isopropyl alcohol of the present invention will be described in detail with reference to the drawings.

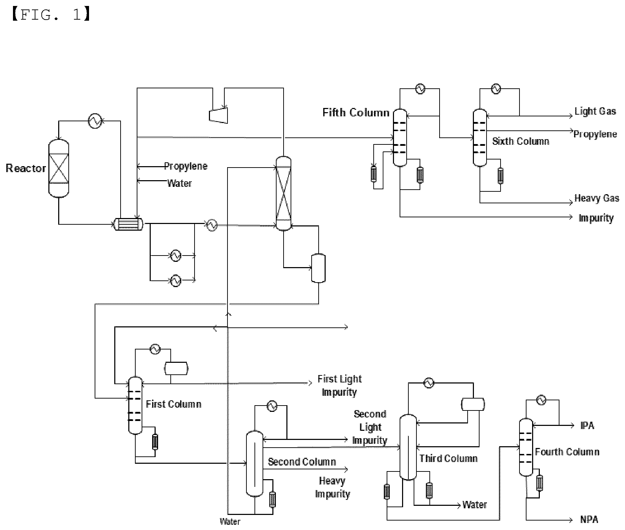

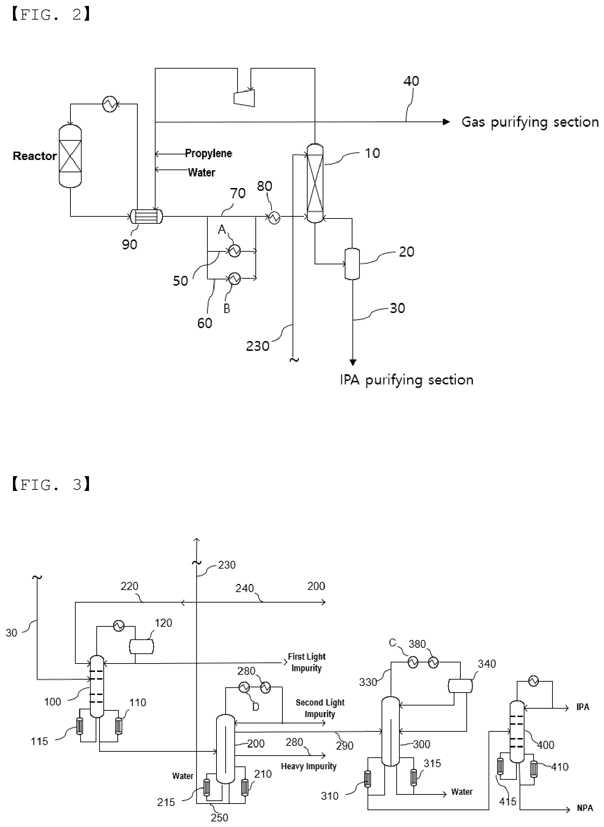

FIG. 1 is a flow diagram of the overall process of a method for preparing isopropyl alcohol according to an embodiment of the present invention. FIG. 2 is a detailed flow diagram showing a process between a reactor and an absorption column in a process of a method for preparing isopropyl alcohol according to an embodiment of the present invention.

Referring to FIGS. 1 and 2, a reaction product including the isopropyl alcohol may be produced by a reaction of propylene and water performed in a reactor. A feed including propylene and water may be supplied to a reactor, and a reaction product produced in the reactor may include isopropyl alcohol, unreacted propylene, unreacted water, and various by-products. In this case, the isopropyl alcohol should be separated and recovered from the reaction product, and an unreacted propylene monomer needs to be recovered and reused in the isopropyl alcohol preparation process.

Specifically, the reactor may be operated under optimal conditions under which isopropyl alcohol may be efficiently prepared through a gas phase reaction of a propylene monomer and water. For example, an operating pressure of the reactor may be 10 kg/cm²·g to 50 kg/cm²·g, 25 kg/cm²·g to 50 kg/cm²·g, or 35 kg/cm²·g to 45 kg/cm²·g, and an operating temperature of the reactor may be 150°C to 220°C, 165°C to 220°C, or 180°C to 215°C. When the reactor is operated at the pressure and temperature in the above ranges, isopropyl alcohol may be effectively produced through the gas phase reaction using propylene monomer and water.

The reaction product produced under the operating conditions of the reactor may be a high-temperature and gaseous reaction product. Meanwhile, the feed including propylene and water, which are reactants required for the reaction, needs to be preheated to a temperature range appropriate for supply to the reactor and then supplied to the reactor. Therefore, according to an embodiment of the present invention, the feed may be first supplied to a heat exchanger 90 and primarily preheated by heat exchange with the reaction product. As the reaction product discharged from the reactor is heat-exchanged with the feed, the high-temperature reaction product discharged from the reactor may be cooled to a level suitable for supplying to an absorption column 10, and at the same time, the feed may be preheated to a level suitable for supplying to the reactor. Through this, heat energy for heating the feed may be saved.

The feed preheated in the heat exchanger 90 may be additionally heated as needed and then supplied to the reactor.

Meanwhile, the propylene included in the feed may include propylene newly supplied for the reaction, propylene supplied from an upper portion of the absorption column 10, and propylene recovered with a high purity from a gas purifying section described below.

Propylene recycled in the process and supplied to the reactor is used again as a raw material for the gas phase reaction, and the propylene after the reaction also needs to be recovered with a high purity in order to prepare high-purity isopropyl alcohol. Specifically, it is preferable that the propylene (C₃H₆) supplied as a raw material to the reactor has a high purity as described above; however, in a case in which the raw material propylene includes other unsaturated hydrocarbons such as ethylene, butene, and pentene, and impurities such as ethane, propane, and carbon dioxide, by-products (for example, ethanol) having a boiling point similar to that of isopropyl alcohol may be produced during the reaction between propylene and water. Therefore, it is preferable that the propylene supplied as a reactant to the reactor includes propylene in an amount of 97 wt% or more, for example, 97 to 99.8 wt%, with respect to the total weight thereof, and a content of impurities is less than 3 wt%.

Meanwhile, only a portion of the propylene supplied to the reactor is used for the reaction. Accordingly, the reaction product may include unreacted propylene and unreacted water in addition to isopropyl alcohol produced by the reaction of the propylene monomer and water. For example, the reaction product may include 65 to 85 wt% of a propylene monomer, 4 to 8 wt% of isopropyl alcohol, and 5 to 30 wt% of water. In addition, the reaction product may include at least two or more types of light by-products and heavy by-products as by-products. Specifically, the reaction product may include diisopropyl ether (DIPE) as a first light by-product, acetone as a second light by-product, and n-propyl alcohol (NPA) and hexanol as heavy by-products. Therefore, there is a need for performing a process of separating unreacted propylene from the reaction product and purifying isopropyl alcohol from various by-products.

Meanwhile, there are various methods for recovering propylene from the reaction product, and in particular, a method for recovering high-purity propylene and recycling the recovered high-purity propylene to the reaction in which the gas phase reaction is performed is presented as an example.

According to an embodiment of the present invention, recovery of propylene in the reaction product may be performed by a gas purifying section including an absorption column, a flash drum, and at least a fifth column and a sixth column. Meanwhile, purification of isopropyl alcohol in the reaction product may be performed by an isopropyl alcohol purifying section including an absorption column, a flash drum, and at least first to fourth columns.

A method for preparing isopropyl alcohol according to an embodiment of the present invention may include cooling the reaction product and supplying the cooled reaction product to the absorption column 10.

Some components in the gaseous reaction product may be condensed and liquefied by the cooling, and other components may be present in gas phases in the reaction product and may be supplied to the absorption column 10. For example, it is preferable that isopropyl alcohol to be discharged through a lower discharge stream of the absorption column 10 is present in a liquid phase, and propylene and gas components to be discharged through an upper discharge stream of the absorption column 10 is present in a gas phase. That is, the efficiency of component separation in the absorption column may be increased by a phase change of some components due to the cooling.

Specifically, when isopropyl alcohol is discharged through an upper portion of the absorption column and then introduced into the reactor again, this has an adverse effect on the isopropyl alcohol production reaction performed in the reactor, and therefore, it is preferable to recover isopropyl alcohol through a lower portion of the absorption column as much as possible. In addition, when propylene and gas components are discharged through the lower portion of the absorption column, an additional gas purifying column is required to recover unreacted propylene or gas components discharged through the lower portion, which increases energy consumption. Therefore, it is preferable to recover these unreacted propylene and gas components through the upper portion of the absorption column as much as possible.

Furthermore, the absorption column absorbs isopropyl alcohol as water introduced through the upper portion of the absorption column flows down the absorption column, and the temperature may be controlled, by the cooling, to a temperature range in which the absorption efficiency of isopropyl alcohol by water is most preferable, and then, the reaction product may be supplied to the absorption column 10. That is, the cooling of the reaction product of the present invention may achieve the effect of increasing the absorption efficiency of isopropyl alcohol in the absorption column 10 through a phase change of some components of the reaction product and temperature control of the reaction product.

From this point of view, the temperature of the cooled reaction product introduced into the absorption column 10 may be 90 to 99°C, and specifically, 90 to 95°C. In this case, the absorption efficiency of isopropyl alcohol by water in the absorption column 100 may be further improved, and propylene discharged through the lower portion of the absorption column may be minimized.

Meanwhile, all or a portion of the reaction product passing through the heat exchanger 90 may be cooled by heat exchange with one or more of a lower discharge stream of a fourth column 400 and a side discharge stream of the fifth column 500.

Referring to FIGS. 2 to 4 according to an embodiment of the present invention, the reaction product is heat-exchanged with one or more of a first reboiler 410 of the fourth column provided below the fourth column 400 of the isopropyl alcohol purifying section and a first reboiler (side reboiler, 510) of the fifth column provided at a side of the fifth column 500 of the gas purifying section, such that energy may be efficiently used.

Specifically, first, as the heat energy of the high-temperature reaction product is supplied to one or more of the fourth column 400 and the fifth column 500, heat energy required for the operation of these columns may be reduced. That is, most of the heat energy required for the operation of one or more of the fourth column 400 and the fifth column 500 may be replaced by utilizing waste heat. Second, the amount of refrigerant required to cool the reaction product in the related art may be reduced. Third, in order to increase the purification efficiency in the fifth column 500 of the gas purifying section, it is essential to implement a gentle temperature profile of the column, and as the heat energy of the reaction product is supplied to a lower and middle portion of the fifth column 500, such that a gentle temperature profile across the lower and middle portion of the fifth column may be implemented.

Meanwhile, according to an embodiment of the present invention, the cooling of the reaction product may be performed by including a first cooling and a second cooling, the first cooling being performed by heat exchange between all or a portion of a reaction product stream and one or more of the lower discharge stream of the fourth column 400 and the side discharge stream of the fifth column 500, and the second cooling being performed by heat exchange between a stream (high-temperature medium) including the reaction product subjected to the first cooling and a refrigerant (low temperature medium).

Specifically, referring to FIG. 2, the reaction product stream may be branched into a first branched stream 50, a second branched stream 60, and a third branched stream 70. Here, the first branched stream 50 may be a stream that is heat-exchanged with the lower discharge stream of the fourth column, and the second branched stream 60 may be a stream that is heat-exchanged with the side discharge stream of the fifth column. Meanwhile, the third branched stream 70 may be a stream that does not exchange heat with the fourth column and the fifth column.

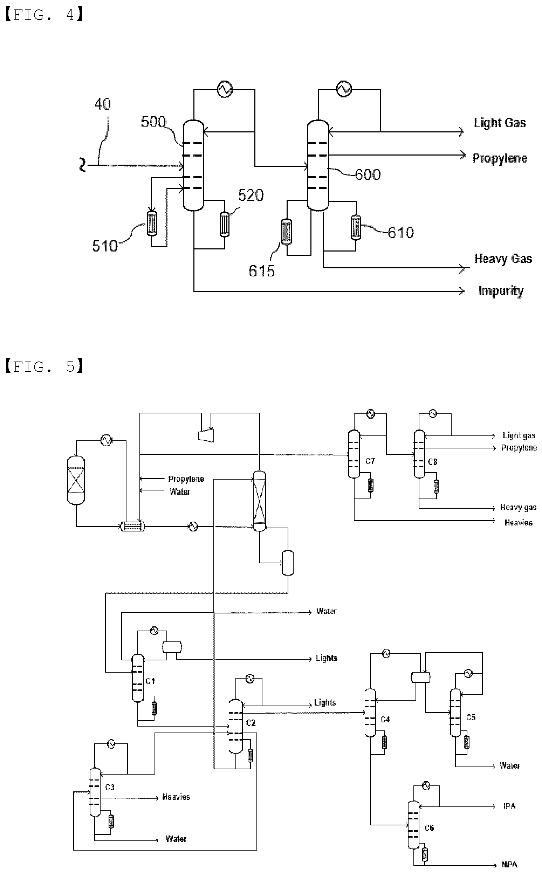

Specifically, the first branched stream 50 may be a stream that is heat-exchanged with the lower discharge stream of the fourth column 400 in the first reboiler 410 of the fourth column. That is, referring to FIGS. 2 and 3, the heat exchanger A of FIG. 2 is the first reboiler 410 of the fourth column. Meanwhile, the second branched stream 60 may be a stream that is heat-exchanged with the side discharge stream of the fifth column 500 in the first reboiler 510 of the fifth column. That is, referring to FIGS. 2 and 4, the heat exchanger B of FIG. 2 is the first reboiler 510 of the fifth column.

In this case, the first cooling may be cooling performed by heat exchange between the first branched stream 50 and the lower discharge stream of the fourth column and heat exchange between the second branched stream 60 and the side discharge stream of the fifth column.

The first and second branched streams 50 and 60 that are subjected to the first cooling are combined with the third branched stream 70 that is not subjected to the first cooling to form a combined stream, and the combined stream may be additionally subjected to the second cooling by a refrigerant in a heat exchanger 80. Here, the refrigerant may be cooling water (CW).

Furthermore, a ratio of mass flow rates of the first to third branched streams, that is, the mass flow rate of the first branched stream 50:the mass flow rate of the second branched stream 60:the mass flow rate of the third branched stream 70, may be 1:0.2 to 0.4:0.4 to 0.7. When the ratio is within the above range, the maximum amount of heat may be supplied relative to the size of the first reboiler 410 of the fourth column and the first reboiler 510 of the fifth column, and the size of an auxiliary reboiler 415 provided below the fourth column 400 and the size of a second reboiler 520 provided below the fifth column 500 may be minimized. Subsequently, the reaction product cooled through the first cooling and second cooling may be supplied to the absorption column 10. Here, the cooled reaction product may be supplied to the absorption column 10 as a gas-liquid mixed phase. In the absorption column 10, a lower discharge stream of the absorption column including isopropyl alcohol and an upper discharge stream of the absorption column including propylene may be separated.

The reaction product may be supplied to the lower portion of the absorption column 10, and water may be supplied to the upper portion of the absorption column 10. Using the water supplied to the upper portion, the isopropyl alcohol included in the reaction product is dissolved and then separated through the lower portion of the absorption column 10 and the stream including propylene may be separated through the upper portion of the adsorption column 10.

The water supplied to the upper portion of the absorption column 10 may be a stream 230 that is branched and supplied from a lower discharge stream of a second column 200 of the isopropyl alcohol purifying section.

The lower discharge stream of the absorption column 10 may be supplied to a flash drum 20, and propylene and gas components that may be present in the lower discharge stream of the absorption column 10 may be separated through an upper portion of the flash drum 20 and re-supplied to the absorption column 10 again. Through this, propylene and gas components may be eliminated or minimized in a lower discharge stream 30 of the flash drum 20. The lower discharge stream 30 of the flash drum 20 may be introduced into the isopropyl alcohol purifying section.

Meanwhile, the upper discharge stream of the absorption column 10 may include propylene and gas components. The upper discharge stream of the absorption column 10 may be compressed by a compressor, and then, a portion of a stream 40 may be supplied to a gas purifying section described below, and the remaining stream may be mixed with propylene and water to be newly supplied, introduced into the heat exchanger 90 as described above, and then recycled to the reactor.

According to an embodiment of the present disclosure, the isopropyl alcohol purifying section may include first to fourth columns. The lower discharge stream of the absorption column, and preferably, the isopropyl alcohol included in the lower discharge stream 30 of the flash drum 20, may be obtained by sequentially passing through a lower discharge stream of the first column, a first side discharge stream of the second column, a first region lower discharge stream of the third column, and an upper discharge stream of the fourth column. Hereinafter, a purification process performed in the isopropyl alcohol purifying section will be described with reference to FIG. 3.

According to an embodiment of the present invention, at least one or more streams of the lower discharge stream of the absorption column 10, the lower discharge stream of the flash drum 20, and the lower discharge streams of the fifth and sixth columns of the gas purifying section may be supplied to the first column as a feed of isopropyl alcohol purifying section. Specifically, a feed stream supplied to the first column may be the lower discharge stream 30 of the flash drum 20. The feed stream supplied to the first column may include isopropyl alcohol, water, a first light by-product, a second light by-product, and a heavy by-product.

The feed stream 30 supplied to the first column may be introduced into a point at a height of 30 to 50% downwards from the top of the first column 100.

According to an embodiment of the present invention, the first light by-product included in the feed 30 may be first separated and removed by a layer separator 120 connected to the first column 100 and an upper portion of the first column 100.

Specifically, an upper discharge stream of the first column including isopropyl alcohol, water, the first light by-product (first light impurity), and, optionally, the second light by-product and a lower discharge stream of the second column including isopropyl alcohol, water, the second light by-product, and the heavy by-product may be discharged through the upper portion and the lower portion of the first column 100, respectively, by the distillation in the first column 100.

The upper discharge stream of the first column may be supplied to a condenser and cooled and liquefied after being discharged from the first column 100. The liquefied upper discharge stream of the first column may be supplied to the layer separator 120 and subjected to liquid-liquid separation. By the liquid-liquid separation, a water phase stream including isopropyl alcohol, water, and optionally the second light by-product may be refluxed to the first column, and an oil phase stream including the first light by-product may be discharged outside the system. The amount of the first light by-product discharged outside the system may be 97 wt% or more, 99 wt% or more, and specifically, 100 wt%, when the content of the first light by-product included in the feed stream 30 supplied to the first column is 100 wt%.

In order for the first light by-product to be easily separated by distillation in the first column 100 and liquid-liquid separation in the layer separator 120 provided above the first column, at least the first light by-product should be an oily component that does not dissolve in water. That is, the first column 100 is operated under operating conditions in which a heavy by-product is not vaporized, and the first light by-product is substantially separated from water and isopropyl alcohol dissolved in water in the layer separator 120, such that the first light by-product may be efficiently separated.

According to an embodiment of the present invention, the first light by-product may be diisopropyl ether (DIPE) which is insoluble in water, and the second light by-product may be acetone which is soluble in water. Since a boiling point of acetone is lower than that of diisopropyl ether, the upper discharge stream of the first column may include water, isopropyl alcohol, acetone, and diisopropyl ether (DIPE). The isopropyl alcohol included in the upper discharge stream of the first column is separated from the first light by-product (oil phase) by liquid-liquid separation performed in the layer separator 120, and water, isopropyl alcohol, and acetone included in the water phase are refluxed back to the first column 100. Therefore, the loss of isopropyl alcohol at the upper portion of the first column 100 may be minimized by the layer separator 120 provided above the first column 100.

Meanwhile, almost all of the first light by-product included in the feed stream 30 supplied to the first column may be discharged outside the system. To this end, the operating conditions of the first column 100 should be controlled so that almost all of the first light by-product included in the feed 30 may be included in the upper discharge stream of the first column 100.

Specifically, an operating temperature of the first column 100 may be 75°C or higher or 80°C or higher and 95°C or lower or 90°C or lower. The operating temperature may refer to a temperature at the lower portion of the first column 100. Meanwhile, an operating pressure of the first column 100 may be 1 kg/cm²·g or less or 0.5 kg/cm²·g or less. The operating pressure may refer to a pressure at the upper portion of the first column 100. When the first column 100 is operated at the operating temperature and the operating pressure as described above, the first light by-product may be separated as much as possible through the upper discharge stream of the first column, and therefore, the first light by-product may be prevented from flowing out through the lower discharge stream of the first column and remaining as an impurity in isopropyl alcohol prepared as a result. Furthermore, the operating temperature and the operating pressure of the first column 100 are as described above, and efficient energy utilization is possible through heat exchange with an upper discharge stream of the third column described below.

Meanwhile, in order to effectively recover the isopropyl alcohol included in the upper discharge stream of the first column and reflux the recovered isopropyl alcohol back to the first column by the liquid-liquid separation performed in the layer separator 120, water should be supplied to the upper portion of the first column. The water supplied to the upper portion of the first column is supplied to the first column separately from the water included in the feed stream 30 supplied to the first column. The water supplied to the upper portion of the first column may be water included in a stream 220 that is branched and recycled from a portion of a lower discharge stream 250 of the second column including water as described below.

Specifically, the stream discharged through the lower portion of the second column 200 may be branched into a reflux stream supplied to the first reboiler 210 of the second column 200, and the remaining stream may be branched into the stream 230 supplied to the upper portion of the absorption column 10, a stream 240 including water discharged outside the system, and the stream 220 recycled to the upper portion of the first column. That is, the branched stream 220 branched from a portion of the lower discharge stream of the second column 200 including water may be a branched stream branched from a portion of the stream 250 after branching the stream that is refluxed to the first reboiler 210 of the second column 200 among the streams immediately after being discharged through the lower portion of the second column 200.

Meanwhile, according to an embodiment of the present invention, a mass flow rate of the branched stream 220 of the lower discharge stream 250 of the second column recycled to the upper portion of the first column needs to be controlled from the viewpoint of the loss of isopropyl alcohol in the layer separator 120 and the energy consumption in the first column 100. Specifically, the mass flow rate of the branched stream 220 of the lower discharge stream 250 of the second column may be 0.4 to 1.2, 0.4 to 1.0, or 0.5 to 0.8, with respect to a mass flow rate of the feed stream 30 supplied to the first column 100. When a flow rate of the water supplied to the upper portion of the first column is larger than 1.2, the energy consumption required in the first column excessively increases. On the other hand, when the flow rate of the water supplied to the upper portion of the first column is less than 0.4, it is difficult to supply sufficient water to the layer separator 120, and thus, the loss of isopropyl alcohol in an oil phase may occur or may increase excessively.

Meanwhile, in the layer separator 120 provided above the first column 100, isopropyl alcohol should be included in the water phase and refluxed together with water to the first column 100. In a case in which the isopropyl alcohol is included in the oil phase, the loss of isopropyl alcohol occurs in the layer separator 120, and in order to prevent the loss of isopropyl alcohol, a sufficient amount of water should be secured in the layer separator 120. The amount of water in the layer separator 120 is affected by the amount of water introduced into the first column 100.

According to an embodiment of the present invention, the water introduced into the first column 100 may be the water included in the feed stream 30 supplied to the first column and the water included in the stream 220 recycled after being branched from a portion of the lower discharge stream 250 of the second column. In order to minimize the loss of isopropyl alcohol in the layer separator 120, it is preferable that the sum of a mass flow rate of the water included in the feed stream 30 and a mass flow rate of the water included in the stream 220 recycled after being branched from a portion of the lower discharge stream 250 of the second column is maintained to be 12 to 15 times (the ratio of the mass flow rates of isopropyl alcohol and water) the mass flow rate of the isopropyl alcohol included in the feed stream 30. In this case, a sufficient amount of water may be supplied to the layer separator 120, the loss of isopropyl alcohol in an oil phase in the layer separator 120 may be prevented, and at the same time, the energy consumption required for the operation of the first column 100 may be optimized.

That is, when a ratio of the mass flow rates of the isopropyl alcohol and water supplied to the first column is less than 12, it is difficult to secure a sufficient amount of water in the layer separator 120, such that the loss of isopropyl alcohol in an oil phase occurs, and it is difficult to achieve a desired recovery rate of isopropyl alcohol. Furthermore, in this case, a portion of the first light by-product that should be removed while included in an oil phase in the layer separator 120 is included in a water phase and introduced into the second column 200, and in a case in which the first light by-product is introduced into the second column 200, the first light by-product is also included in the first side discharge stream of the second column including a mixture of isopropyl alcohol and water, and the purity of isopropyl alcohol recovered in the second column is consequently reduced.

In addition, a ratio of the mass flow rates of isopropyl alcohol and water supplied to the first column is larger than 15, the loss of isopropyl alcohol in the layer separator 120 may be prevented, but the amount of water recycling through the first column 100 and the second column 200 is excessively large, which may increase the energy consumption in the two columns.

Furthermore, from the viewpoint of minimizing the loss of isopropyl alcohol in the layer separator 120 and reducing the energy consumption in the first and second columns, the mass flow rate of the water included in the stream 220 recycled after being branched from a portion of the lower discharge stream 250 of the second column may be 58% to 90% based on the mass flow rate of the water included in the feed stream 30.

Meanwhile, a first reboiler 110 supplying heat energy required for the operation of the first column is provided below the first column 100. A reflux stream of the lower discharge stream of the first column may be introduced into the first reboiler 110 of the first column, heat-exchanged with a high-temperature heat source, and then introduced again into the lower portion of the first column 100. Heat energy required for the operation of the first column 100 may be supplied to the first column 100 through the first reboiler 110 of the first column.

According to an embodiment of the present invention, the heat source of the first reboiler 110 of the first column, that is, the heat source that is heat-exchanged with the reflux stream of the lower discharge stream of the first column, may be an upper discharge stream 330 of the third column 300, as described below. Specifically, all or a portion of the upper discharge stream 330 of the third column may be heat-exchanged with the reflux stream of the lower discharge stream of the first column in the first reboiler 110 of the first column before being introduced into a condenser 380 or a layer separator 340 provided above the third column 300. Through this, the heat energy of the upper discharge stream 330 of the third column 300 may be supplied to the first column 100.

Meanwhile, according to an embodiment of the present invention, in a case in which the heat energy of the upper discharge stream 330 of the third column 300 alone cannot completely replace the reboiler duty required for the operation of the first column 100, an auxiliary reboiler 115 may be provided below the first column 100 separately from the first reboiler 110 of the first column.

According to an exemplary embodiment of the present invention, the lower discharge stream of the first column is introduced into the second column 200, and the second light by-product, the mixture of isopropyl alcohol and water, the heavy by-product, and water may be separated according to boiling point.

Specifically, a step of supplying the lower discharge stream of the first column including the isopropyl alcohol, water, second light by-product, and heavy by-product to the second column 200 and separating the lower discharge stream of the first column into each of an upper discharge stream of the second column including the second light by-product (second light impurity), a first side discharge stream 290 of the second column including the mixture of isopropyl alcohol and water, a second side discharge stream 280 of the second column including the heavy by-product, and a lower discharge stream 250 of the second column including the water may be performed.

The second light by-product is a by-product having the relatively lowest boiling point compared to other separated components, and the second light by-product may be a compound having a boiling point of 50 to 70°C, and specifically, may be acetone. The acetone may be a by-product produced during a gas phase reaction for preparing isopropyl alcohol, and may be a by-product produced by oxidation of isopropyl alcohol in a subsequent process after the gas phase reaction. The upper discharge stream of the second column may include 60 wt% or more, 70 wt% or more, or 90 wt% or more, and 100 wt% or less of the second light by-product, and may include the residual mixture of isopropyl alcohol and water. After the upper discharge stream of the second column is discharged from the second column, a portion of the upper discharge stream of the second column may be refluxed back to the second column after passing through the condenser, and the remainder may be discharged outside the system.

Meanwhile, according to an embodiment of the present invention, the upper discharge stream of the second column 200 may be heat-exchanged with a lower discharge stream of the sixth column. Specifically, referring to FIGS. 3 and 4, the upper discharge stream of the second column 200 may be heat-exchanged with the lower discharge stream of the sixth column 600 in a first reboiler 610 of the sixth column provided below the sixth column 600 before being introduced into the condenser 280 provided above the second column 200. In this case, a heat exchanger D provided above the second column 200 may be the first reboiler 610 of the sixth column. Through this, the heat energy of the upper discharge stream of the second column 200 may be supplied to the sixth column 600. The upper discharge stream of the second column 200 that is heat-exchanged with the lower discharge stream of the sixth column 600 may be additionally refluxed to the second column 200 or discharged outside the system after passing through the condenser 280 provided above the second column 200.

Meanwhile, the mixture of isopropyl alcohol and water may be an azeotrope of isopropyl alcohol and water. That is, water having a boiling point of about 100°C and isopropyl alcohol having a boiling point of about 82.3°C may form an azeotrope at an azeotropic temperature of about 81°C. A boiling point of the azeotrope of isopropyl alcohol and water is higher than a boiling point of the second light by-product and is lower than a boiling point of the heavy by-product.

Therefore, a portion of the water introduced into the second column forms an azeotrope with isopropyl alcohol and is discharged through the first side discharge stream 290 of the second column, and the remaining water is discharged through the lower discharge stream 250 of the second column.

The first reboiler 210 of the second column supplying heat energy required for the operation of the second column is provided below the second column 200. A reflux stream of the lower discharge stream of the second column may be introduced into the first reboiler 210 of the second column, heat-exchanged with a high-temperature heat source, and then introduced again into the lower portion of the second column 200. Heat energy required for the operation of the second column 200 may be supplied to the second column 200 through the first reboiler 210 of the second column.

According to an embodiment of the present invention, the heat source of the first reboiler 210 of the second column, that is, the heat source that is heat-exchanged with the reflux stream of the lower discharge stream of the second column, may be the upper discharge stream 330 of the third column 300, as described below. Specifically, all or a portion of the upper discharge stream 330 of the third column may be heat-exchanged with the reflux stream of the lower discharge stream 250 of the second column in the second reboiler 230 before being introduced into the condenser 380 or the layer separator 340 provided above the third column 300. Through this, the heat energy of the upper discharge stream of the third column 300 may be supplied to the first column 100.

According to an embodiment of the present invention, in a case in which the heat energy of the upper discharge stream 330 of the third column 300 alone cannot completely replace the reboiler duty required for the operation of the second column 200, an auxiliary reboiler 215 may be provided below the second column 200 separately from the first reboiler 210 of the second column.

Meanwhile, the lower discharge stream 250 of the second column including water, specifically, the branched stream 220 branched from a portion of the stream 250 that is not supplied to the reboiler 210 among the streams discharged through the lower portion of the second column 200, may be recycled to the upper portion of the first column. The water recycled from the second column 200 may be used to supplement a sufficient amount of water so that the phase separation between the water phase and the oil phase may be smoothly performed in the layer separator 120 provided above the first column 100.

Meanwhile, the heavy by-product may include n-propyl alcohol (NPA) and hexanol, and these heavy by-products may be discharged through the second side discharge stream 280 of the second column.

According to an embodiment of the present invention, the second column 200 includes a dividing wall spaced apart from the bottom and provided in a longitudinal direction of the column, and the second column may be a distillation column partitioned into a top region, a bottom region, a supply region, and a discharge region by the dividing wall.

Referring to FIG. 3, the top region is a region located above an upper end of the dividing wall and is a region from which the upper discharge stream of the second column is discharged, and the bottom region is a region located below a lower end of the dividing wall and is a region from which the lower discharge stream 250 of the second column is discharged. Meanwhile, the lower discharge stream of the first column may be supplied to the supply region.

The first side discharge stream 290 of the second column and the second side discharge stream 280 of the second column may be discharged from the discharge region among the regions partitioned by the dividing wall. Specifically, the first side discharge stream 290 may be discharged from the discharge region at a higher position than the second side discharge stream 280.

That is, according to an embodiment of the present invention, the composition (the lower discharge stream of the first column) including at least four components such as isopropyl alcohol, water, a second light by-product, and a heavy by-product is separated and discharged through the upper portion, the first side, the second side, and the lower portion of one column (the second column) provided with a dividing wall, such that the number of distillation columns previously required for separation of these components may be reduced.

Specifically, referring to FIG. 5 in which a distillation column without a dividing wall is used as a C2 column, although it is not impossible to discharge the separated material through an upper portion, a lower portion, a first side, and a second side of the C2 column in FIG. 5, in particular, a side discharge stream through which a heavy by-product is separated includes a large amount of isopropyl alcohol and water; thus, additional purification of the side discharge stream is required to increase a yield of isopropyl alcohol. That is, it is required to perform a process of introducing the side discharge stream into a C3 column, additionally recovering a stream including isopropyl alcohol to the upper portion, and supplying the recovered stream again to the C2 column. That is, referring to FIG. 5, the same roles performed by the conventional C2 column and C3 column may be performed by one distillation column provided with a dividing wall, such that the number of columns may be reduced, and the energy consumption (for example, steam consumption) required for the operation of the column may be reduced, and as the second column provided with a dividing wall is used, the preliminary separation is performed in the supply region and the final purification is performed in the discharge region, such that energy consumption may be reduced more than by simply combining two columns.

Meanwhile, the upper end of the dividing wall may be located at a height of 3% to 30% downwards from the top of the second column 200, and the lower end of the dividing wall may be located at a height of 70% to 95% downwards from the top of the second column 200.

Furthermore, the first side discharge stream 290 may be discharged at a height of 5 to 33% downwards from the top of the second column, and the second side discharge stream 280 may be discharged at a height of 40 to 80% downwards from the top of the second column.

Through the location of the dividing wall and the discharge points of the first and second side discharge streams, the energy consumption may be reduced compared to when using two conventional columns, and at the same time, four streams discharged from the second column may be obtained with a desired purity.

An operating temperature and an operating pressure of each of the top region and the bottom region of the second column 200 also need to be controlled from the viewpoint of the purity of the separated components such as isopropyl alcohol and the energy required to separate these components.

Specifically, the operating temperature of the top region of the second column 200 may be 90°C or lower, 85°C or lower, or 80°C or lower, and the operating pressure of the top region may be 2 kg/cm²·g or less, 1 kg/cm²·g or less, or 0.05 kg/cm²·g or less. Meanwhile, the operating temperature of the bottom region of the second column 200 may be 85°C or higher or 88°C or higher and 105°C or lower or 103°C or lower. Meanwhile, the operating pressure of the bottom region may be 1.0 kg/cm²·g or less or 0.5 kg/cm²·g or less.

When the operating temperature and the operating pressure of the second column 200 are as described above, efficient energy may be utilized by heat exchange between the lower discharge stream of the second column and the upper discharge stream of the third column performed in the first reboiler 210 of the second column, and at the same time, efficient energy may be utilized by heat exchange between the lower discharge stream of the sixth column and the upper discharge stream of the second column performed in the first reboiler 610 of the sixth column.

The method for preparing isopropyl alcohol according to an embodiment of the present invention may include: supplying the first side discharge stream 290 of the second column including the mixture of isopropyl alcohol and water to the third column 300 and performing azeotropic distillation in the presence of an azeotropic agent.

The third column 300 may be a dividing wall distillation column that includes a dividing wall that is connected to the bottom and extends in a longitudinal direction of the column, and is partitioned, by the dividing wall, into a first region, a second region facing the first region, and an upper region located above an upper end of the dividing wall. Specifically, the dividing wall may be connected (coupled) to the bottom of the third distillation column 300 and may be provided to extend upward in the longitudinal direction of the third distillation column 300. In this case, the first region and the second region are regions partitioned so as to face each other with the dividing wall therebetween, and the second region is a region facing the first region. Meanwhile, the upper region is a region located above the upper end of the dividing wall.

Specifically, the first side discharge stream 290 of the second column 200 may include a mixture of isopropyl alcohol and water, and specifically, an azeotrope of isopropyl alcohol and water. More specifically, the first side discharge stream 290 of the second column may include 80 to 90 wt% of isopropyl alcohol and 10 to 20 wt% of water.

Meanwhile, azeotropic distillation may be performed in the presence of an azeotropic agent in the third distillation column 300. A portion of the isopropyl alcohol and a portion of the water included in the first side discharge stream 290 of the second column 200 may form an azeotrope. Since the azeotrope cannot be completely separated into these components by general distillation, an azeotropic relationship between isopropyl alcohol and water may be removed using a general azeotropic agent, and then, isopropyl alcohol and water may be separated with a high purity. The azeotropic agent of the present invention performing these functions may be one or more selected from the group consisting of cyclohexane, benzene, toluene, and isopropyl acetate.

The azeotropic agent is a substance added separately from the feed component for azeotropic distillation, but the azeotropic agent is an impurity in terms of isopropyl alcohol or the like, and therefore, the azeotropic agent should be separated through a separate distillation column or the like, and from an economic perspective, the separated azeotropic agent should be recyclable.

That is, referring to FIG. 5 regarding the related art, in the related art, in order to separate isopropyl alcohol and water from a feed including an azeotrope of isopropyl alcohol and water, and by-products, azeotropic distillation was performed in a general azeotropic distillation column C4 not provided with a dividing wall in the presence of an azeotropic agent, an upper discharge stream including water and the azeotropic agent was phase-separated in a layer separator, and then an oil phase including the azeotropic agent was refluxed back to the azeotropic distillation column C4. However, a water phase includes a large amount of the azeotropic agent in addition to water, and therefore, the water phase is introduced into an azeotropic agent recovery column C5 to separate the azeotropic agent and water by distillation, the recovered azeotropic agent is introduced again into the azeotropic distillation column C4, and the water is discharged outside the system. Here, in order to separate the azeotropic agent and water by distillation in the azeotropic agent recovery column C5, a large amount of energy had to be supplied through the reboiler provided below the azeotropic agent recovery column C5.

Meanwhile, according to the related art, the azeotropic distillation column C4 was supplied with heat energy required for the operation of the azeotropic distillation column C4 by a reboiler provided below the azeotropic distillation column C4. In a case in which water and an azeotropic agent were included in an upper discharge stream of the azeotropic distillation column C4, isopropyl alcohol and by-products were included in a lower discharge stream of the azeotropic distillation column C4, and a process of supplying the lower discharge stream of the azeotropic distillation column C4 to an isopropyl alcohol recovery column C6 to obtain isopropyl alcohol through an upper portion of the isopropyl alcohol recovery column C6 and separate the by-products through a lower portion of the isopropyl alcohol recovery column C6 was performed.

However, referring to FIGS. 1 and 3 regarding the method for preparing isopropyl alcohol according to an embodiment of the present invention, the present invention is designed to provide the dividing wall in the third distillation column 300 in which azeotropic distillation is performed and to optimize the reflux points of the water phase and oil phase refluxed in the layer separator 340, such that high-purity isopropyl alcohol may be obtained even without providing the azeotropic agent recovery column C5 for separating the azeotropic agent and water as in the related art. Through this, the heat energy supplied to the reboiler for the operation of the azeotropic agent recovery column C5 in the related art may be saved, and the cooling energy required for the operation of the condenser provided above the azeotropic agent recovery column C5 for the operation of the azeotropic agent recovery column C5 may be saved. Furthermore, in addition to energy savings due to non-operation of the azeotropic agent recovery column C5, the energy consumption required for the operation of the distillation column may be reduced even when comparing the third distillation column 300 of the present invention, which is a dividing wall distillation column, with the azeotropic distillation column C4 and distillation columns of the related art.

To this end, according to an embodiment of the present invention, the first region provided at the lower portion of the third distillation column 300 may be provided with a reboiler 310 connected to a lower portion of the first region, and the second region may be provided with a reboiler 315 connected to a lower portion of the second region. Here, the lower portion refers to a point at a height of 90% to 100% downwards from the top (uppermost) of the third distillation column 300. The first and second regions may be supplied with heat energy through the reboilers 310 and 315, respectively, and the operating conditions of the first and second regions may be controlled by controlling the heat energy supplied to each of the reboilers 310 and 315.

According to an embodiment of the present invention, the heat energy supplied through the reboiler 310 connected to the lower portion of the first region may be 1.5 to 3 times, and more specifically, 1.8 to 2.5 times the heat energy supplied through the reboiler 315 connected to the lower portion of the second region. Through this, isopropyl alcohol with a desired purity may be separated through a lower discharge stream from the first region of the third distillation column, and pure water may be separated through a lower discharge stream from the second region.

A temperature of the lower portion of the first region may be 135°C or higher or 137°C or higher and 150°C or lower or 148°C or lower. In addition, a temperature of the lower portion of the second region 302 may be 155°C or higher or 158°C or higher and 170°C or lower or 168°C or lower. Here, each temperature of the lower portion is an operating temperature at a height of 90% to 100% downwards from the top (uppermost) of the column 300 in the first and second regions. By controlling the temperatures of the lower portions of the first and second regions as described above, the energy required for distillation in the third distillation column 300 may be reduced, isopropyl alcohol with a desired purity may be obtained from the lower portion of the first region, and pure water may be separated from the lower portion of the second region.

Meanwhile, the first region lower discharge stream discharged from the first region of the third distillation column 300 may include isopropyl alcohol and a heavy by-product. Here, the heavy by-product may include n-propyl alcohol (NPA). A portion of the first region lower discharge stream may be heat-exchanged in the reboiler 310 connected to the lower portion of the first region and then refluxed back to the first region, and the remainder of the first region lower discharge stream may be supplied to the fourth column 400.

Meanwhile, the second region lower discharge stream discharged from the second region may include water. A portion of the second region lower discharge stream may be heat-exchanged in the reboiler 315 connected to the lower portion of the second region and then refluxed back to the second region, and the remainder of the second region lower discharge stream may be discharged outside the system.

As described above, a region located above the upper end of the dividing wall in the third distillation column 300 may form an upper region. The dividing wall may extend from the bottom, and the upper end of the dividing wall may be located at a height of 10 to 45%, or specifically, at a height of 15 to 30%, downwards from the top of the third distillation column 300. Through this, the separation efficiency of the third distillation column 300 may be maximized, such that isopropyl alcohol with a desired purity may be obtained from the lower portion of the first region, and pure water may be separated from the lower portion of the second region.

The upper discharge stream 330 including water and an azeotropic agent may be discharged from an upper portion of the upper region.

According to an embodiment of the present invention, the upper discharge stream 330 of the third column may be heat-exchanged with one or more of the lower discharge stream of the first column and the lower discharge stream of the second column in one or more of the first reboiler 110 of the first column 100 and the first reboiler 210 of the second column 200. Through this, the heat of the upper discharge stream 330 of the third column is supplied to one or more of the first column and the second column, such that the heat energy required for the operation of one or more of the first column and the second column may be reduced.

That is, referring to FIG. 3, a heat exchanger C provided above the third column may be one or more of the first reboiler 110 of the first column and the first reboiler 210 of the second column. The upper discharge stream 330 of the third column may be discharged from the third column 300 and then transferred to one or more of the first reboiler 110 of the first column and the first reboiler 210 of the second column, and the retained heat energy may be supplied to one or more of the first column 100 and the second column 200. In this case, the upper discharge stream 330 of the third column may be heat-exchanged in the first reboiler 110 of the first column or the first reboiler 210 of the second column, or may be heat-exchanged in both the first reboiler 110 of the first column and the first reboiler 210 of the second column. In the case in which the upper discharge stream 330 of the third column is heat-exchanged in both the first reboiler 110 of the first column and the first reboiler 210 of the second column, the upper discharge stream 330 of the third column may be branched into two streams, and the branched streams may be heat-exchanged in the first reboiler 110 of the first column and the first reboiler 210 of the second column, respectively. In this case, the heat exchanger C in FIG. 1 may refer to both the first reboiler 110 of the first column and the first reboiler 210 of the second column.

After the upper discharge stream 330 of the third column is heat-exchanged with one or more of the first reboiler 110 of the first column and the first reboiler 210 of the second column, the heat-exchanged upper discharge stream 330 of the third column may be transferred to the condenser 380 provided above the third column 300. In the case in which the upper discharge stream 330 of the third column is branched and heat-exchanged with both the first reboiler 110 of the first column and the first reboiler 210 of the second column, the respective branched streams may be combined after heat exchange and transferred to the condenser 380.

An operating pressure of the upper portion of the third column 300 may be 4.9 bar.g to 5.1 bar.g. When the operating pressure of the upper portion of the third column is 4.9 bar.g or higher, a temperature of the upper discharge stream 330 of the third column may be higher than a temperature of the lower portion of each of the first and second columns by at least 10°C or more; thus, it is possible to maintain a temperature difference for heat exchange in the first reboiler 110 of the first column and the first reboiler 210 of the second column.

In particular, when the operating pressure of the upper portion of the third column 300 is higher than 5.1 bar.g, a high temperature of the upper discharge stream 330 of the third column may be implemented, and therefore, there is no problem in supplying heat to the first column 100 and the second column 200, but the separation performance performed in the third column 300 is deteriorated. In this case, additional energy should be supplied to the third column 300 to separate components with a desired purity, which is not preferable from the viewpoint of reducing the energy consumption. On the other hand, when the operating pressure of the upper portion of the third column 300 is lower than **4.9** bar.g, it is difficult to maintain the temperature of the upper discharge stream 330 of the third column at a high temperature, and therefore, it is difficult to secure a sufficient temperature difference to enable heat exchange in the first reboiler 110 of the first column and the first reboiler 210 of the second column.

Meanwhile, the operating temperature of the upper portion of the third column 300, specifically, the operating temperature of the upper portion of the upper region of the third column 300, may be 120°C or higher, and specifically, 122°C or higher. In this case, since an appropriate temperature difference (for example, a temperature difference of at least 10°C or more) required for heat exchange with the lower portions of the first column 100 and the second column 200 may be secured, energy may be efficiently supplied to the first column 100 and the second column 200 by the upper discharge stream 330 of the third column.

Furthermore, in the case in which the upper discharge stream 330 of the third column is heat-exchanged in both the first reboiler 110 of the first column and the first reboiler 210 of the second column, the upper discharge stream 330 of the third column may be branched to form a branched stream supplied to the first reboiler 110 of the first column and a branched stream supplied to the first reboiler 210 of the second column. In this case, a ratio of a mass flow rate of the branched stream supplied to the first reboiler 110 of the first column and a mass flow rate of the branched stream supplied to the first reboiler 210 of the second column may be 1:5 to 1:7. In the case of the above ratio, a logarithmic mean temperature difference (LMTD) between a low temperature medium (the lower discharge stream of each of the first and second columns) and a high temperature medium (the upper discharge stream of the third column) that are heat-exchanged in the first reboiler 110 of the first column and the first reboiler 210 of the second column may be maximized, such that the first reboiler 110 of the first column and the first reboiler 210 of the second column may be efficiently designed and operated. Furthermore, the size of the auxiliary reboilers 115 and 215 of the first and second columns described above may be minimized.

The upper discharge stream 330 of the third column 300 may be heat-exchanged with one or more of the lower discharge stream of the first column and the lower discharge stream of the second column in one or more of the first reboiler 110 of the first column and the first reboiler 210 of the second column, and then, all or a portion thereof may be condensed into a liquid phase while being cooled. The condensed upper discharge stream 330 of the third column 300 may be introduced into the condenser 380 provided above the third column and additionally condensed to a desired condensation condition.

A stream discharged from the condenser 380 subjected to the additional condensation may be introduced into the layer separator 340 provided above the third column. The layer separator 340 is a device that separates a fluid by density difference, and the fluid may be separated into a water phase including water and an oil phase including an azeotropic agent by the layer separator 340. The oil phase including an azeotropic agent may be refluxed to the upper region, and the water phase including water may be refluxed to the second region.

That is, after the upper discharge stream 330 is separated into an oil phase and a water phase by the layer separator 340, the separated oil phase and water phase are refluxed back to the third distillation column 300; thus, a mass flow rate of the upper discharge stream 330 of the third distillation column may be equal to the sum of mass flow rates of a water phase stream and an oil phase stream that are refluxed to the third distillation column 300 through the layer separator 340. That is, the upper discharge stream 330 of the third distillation column 300 is discharged from the third column 300, supplied to one or more of the first reboiler 110 of the first column and the first reboiler 210 of the second column, supplied to the condenser 380, passed through the layer separator 340, and then, entirely refluxed to the third distillation column 300.

The azeotropic agent included in the oil phase stream is used again for azeotropic distillation performed in the third distillation column 300. In addition, when the separated oil phase and water phase are all refluxed to the third distillation column 300 and the reflux point is optimized, the first region lower discharge stream and the second region lower discharge stream may be separated with a high purity.

The reflux point of the water phase to the second region may be a point corresponding to a height of 10% to 50%, and specifically, 25% to 40%, of the dividing wall from the bottom. Through this, energy required for distillation may be minimized, and at the same time, pure water may be separated from the lower portion of the second region.

More specifically, when the position of the upper end of the dividing wall and the reflux point of the water phase to the second region are set as described above, an azeotropic distillation region for separating isopropyl alcohol and water using an azeotropic agent and a distillation region for purifying isopropyl alcohol may be sufficiently secured simultaneously within the third distillation column 300. Furthermore, as the first and second regions share the upper region of the dividing wall, the energy consumption required in the condenser 380 may be reduced, and as the liquid phase reflux stream branched from the lowest portion of the upper region into the first region and the second region and flowing downwards is optimized and distributed, the heat required in the reboiler in each region may be minimized.

Meanwhile, according to an embodiment of the present invention, the first region lower discharge stream discharged from the first region may be supplied to the fourth column 400 for recovering isopropyl alcohol. The first region lower discharge stream may include isopropyl alcohol and a small amount of heavy by-product, isopropyl alcohol may be finally obtained from the upper portion of the fourth column 400, and the heavy by-product may be separated through the lower portion of the fourth column.

The fourth column 400 may include the first reboiler 410 of the fourth column located below, and as described above, the lower discharge stream of the fourth column may be heat-exchanged with the reaction product stream before being introduced into the absorption column in the first reboiler 410 of the fourth column. Specifically, referring to FIGS. 2 and 3, the first branched stream 50 among the branched streams of the reaction product streams may be heat-exchanged with the lower discharge stream of the fourth column in the first reboiler (410, simultaneously, the heat exchanger A in FIG. 2) of the fourth column.

Meanwhile, in a case in which the heat energy of the first branched stream 50 alone cannot completely replace the reboiler duty required for the operation of the fourth column 400, the auxiliary reboiler 415 may be provided below the fourth column 400 separately from the first reboiler 410 of the fourth column.

Meanwhile, as described above, the upper discharge stream of the absorption column 10 may include propylene and gas components, and after the upper discharge stream of the absorption column 10 is compressed by a compressor, a portion of the stream 40 may be supplied to the gas purifying section described below. Hereinafter, the recovery process performed in the gas purifying section will be described with reference to FIGS. 1 and 4.

The stream supplied to the gas purifying section may be specifically supplied as the feed stream 40 of the fifth column 500 of the gas purifying section. The feed stream 40 of the fifth column 500 includes propylene and gas components, and the gas components include light gas components having a boiling point lower than that of propylene and heavy gas components having a boiling point higher than that of propylene. In addition, the feed stream 40 of the fifth column 500 may include isopropyl alcohol, water, a light by-product such as diisopropyl ether, and a heavy by-product such as n-propyl alcohol that are not yet separated through the lower portion of the absorption column 100, although in small quantities. Propylene recycled to the reactor through the fifth column 500 may be recovered with a higher purity, and at the same time, isopropyl alcohol that may be lost through the upper portion of the fifth column 500 may be entirely recovered to the lower portion.

As described above, the side stream of the fifth column 500 may be heat-exchanged with all or a portion of the reaction product supplied to the absorption column 10. To this end, the fifth column 500 may include the first reboiler 510 of the fifth column 500 connected to the side of the fifth column 500. That is, heat exchange between the reaction product and the side stream of the fifth column 500 may be performed in the first reboiler 510 of the fifth column 500. Specifically, referring to FIGS. 2 and 4, the second branched stream 60 among the branched streams of the reaction product streams may be heat-exchanged with the side discharge stream of the fifth column 500 in the first reboiler (510, simultaneously, the heat exchanger B in FIG. 2) of the fifth column.

More specifically, the side discharge stream of the fifth column 500 may be discharged at a height of 55 to 85% downwards from the top of the fifth column 500 and heat-exchanged with the reaction product. For example, when the side discharge stream is discharged at a height of less than 55%, there is a problem that a section of a contact zone where a specific component is vaporized, and at the same time, the specific component comes into contact with a liquid phase component to be refluxed, that is, a section of a distillation zone where actual distillation and purification are performed is shortened. Meanwhile, when the side discharge stream is discharged at a height exceeding 85%, a temperature of the side discharge stream is excessively high, which makes it difficult to perform heat exchange with the reaction product or to obtain the effect of heat exchange (cooling of the reaction product and heating of the side stream).

Meanwhile, the side discharge stream of the fifth column 500 that is heat-exchanged with the reaction product in the first reboiler 510 of the fifth column may be re-supplied to a stage including the height of the fifth column 500 from which the side stream is discharged.

In addition, the temperature of the side discharge stream discharged from the fifth column 500 may be 40°C to 80°C or 40 to 60°C, and a temperature of the side discharge stream supplied again to the fifth column 500 after heat exchange may be 80°C to 100°C or 85 to 95°C. Through this, a temperature profile according to the height of the fifth column 500 may be appropriately controlled. Therefore, the heat energy required in the fifth column 500, which has been supplied only by the second reboiler 520 in the related art, may be replaced to the maximum extent, more specifically, by more than half.

Meanwhile, according to an embodiment of the present invention, an operating pressure of the upper portion of the fifth column 500 may be a high pressure of 15 kg/cm²·g to 20 kg/cm²·g or 16 kg/cm²·g to 19 kg/cm²·g. In addition, an operating temperature of the lower portion of the fifth column 500 may be 140°C to 180°C or 150°C to 180°C. The separation efficiency of the fifth column 500 is improved within the above ranges of the operating temperature and operating pressure of the fifth column 500, such that isopropyl alcohol, water, and by-products may be entirely recovered to the lower portion of the fifth column 500. Furthermore, inexpensive cooling water may be used as a cooling heat source in the condenser provided above the fifth column 500.

Meanwhile, in a case in which the reaction product and the side discharge stream of the fifth column 500 are heat-exchanged by the first reboiler 510 of the fifth column according to an embodiment of the present invention, it is possible to implement a gentle temperature profile according to height of the fifth column 500, and it is possible to obtain the effect of increasing the section where the temperature according to height of the fifth column 500 changes, that is, the distillation zone in which distillation and purification may be performed. That is, the stream introduced into the fifth column 500 includes not only light components such as propylene and inert gases, but also heavy components such as isopropyl alcohol and water. That is, since the stream introduced into the fifth column 500 includes components having a large difference in boiling point, the temperature profile in the fifth column 500 generally changes rapidly in a specific section, and in this case, there is a zone where the temperature change according to the height of the column is small and component separation is difficult to perform (so-called dead zone). However, a gentle temperature profile is implemented in the fifth column 500 through heat exchange by the first heat exchanger 510 of the fifth column of the present invention, that is, an appropriate temperature gradient according to the height of the column is implemented, such that the zone where component separation is difficult to perform may be converted into a zone where component separation by stripping is possible, thereby increasing the component separation efficiency of the fifth column 500.

In addition, in the case of operating the fifth column 500 with only one lower reboiler as shown in FIG. 5 according to the related art, for the lower reboiler, a high-temperature (for example, 160°C or higher) high-grade heat source (for example, steam) was required. However, in the case in which the first reboiler 510 of the fifth column 500 is provided in the fifth column 500 as in an embodiment of the present invention, the first reboiler 510 of the fifth column 500 may be operated by utilizing low-grade waste heat of about 120°C, and at the same time, the amount of high-grade heat source required for the second reboiler 520 of the fifth column 500 may be reduced compared to the related art, thereby reducing the amount of heat energy used.

Meanwhile, the fifth column 500 may further include the second reboiler 520 connected to the lower portion of the fifth column 500 in addition to the first reboiler 510. The heat energy supplied to the fifth column by the first reboiler 510 of the fifth column may be 50% to 90% of the total heat energy supplied to the fifth column 500 by the first reboiler 510 of the fifth column and the second reboiler 520 of the fifth column.

The upper discharge stream of the fifth column 500 may include propylene, light gas components, and heavy gas components, and the upper discharge stream of the fifth column 500 may be supplied to the sixth column 600 after passing through the condenser.

In the sixth column 600, a portion of the side discharge stream of the sixth column including propylene may be recycled to the reactor, and the remainder may be purged and discharged outside the system. The gas phase component including the inert gas in the upper discharge stream of the sixth column 600 is purged to separate and remove a portion or all of the inert gas, such that a content of the inert gas in the stream refluxed to the reactor is reduced, thereby preventing the inert gas from accumulating in the process.

Meanwhile, as described above, the lower discharge stream of the sixth column 600 may be heat-exchanged with the upper discharge stream of the second column 200 in the first reboiler (610; simultaneously, the heat exchanger D in FIG. 3) of the sixth column provided below the sixth column 600. Through this, the heat energy of the upper discharge stream of the second column 200 may be supplied to the sixth column 600. Furthermore, in a case in which the heat energy of the upper discharge stream of the second column 200 alone cannot completely replace the reboiler duty required for the operation of the sixth column 600, an auxiliary reboiler 615 may be provided below the sixth column 600 separately from the first reboiler 610 of the sixth column.

Hereinafter, the present invention will be described in more detail with reference to examples. However, the following examples are provided for illustrating the present invention. It is apparent to those skilled in the art that various modifications and alterations may be made without departing from the scope and spirit of the present invention, and the scope of the present invention is not limited thereto.

In the following examples and comparative examples, the method according to the present invention was simulated using the commercial process simulation program Aspen Plus V12.1.

### Example 1

A process for preparing isopropyl alcohol was performed according to the process flow of FIG. 1 and FIGS. 2 to 4.

Specifically, water and propylene were supplied to a reactor and subjected to a gas phase reaction to produce a reaction product including isopropyl alcohol, water, and propylene. The reaction product was passed through a heat exchanger 90 and branched into the first to third branched streams. A ratio of mass flow rates of these first to third branched streams was 1:0.33:0.57. The first branched stream 50 was heat-exchanged with a lower discharge stream of a fourth column 400 in a first reboiler 410 of the fourth column, and the second branched stream 60 was heat-exchanged with a side discharge stream of a fifth column 500 in a first reboiler 510 of the fifth column. Each of the heat-exchanged branched streams was combined with the third branched stream 70 and supplied to an upper portion of an absorption column 10.

At this time, the heat energy required for the operation of the fourth column was completely replaced by the heat energy supplied from the first branched stream, and thus, there was no need to supply a separate heat source to an auxiliary reboiler 415 for the operation of the fourth column.

Meanwhile, after a lower discharge stream of the absorption column 10 was supplied to a flash drum 20, a lower discharge stream of the flash drum 20 was supplied to a first column 100 in an isopropyl alcohol purifying section including first to fourth columns. An upper discharge stream of the absorption column 10 was compressed by a compressor and then a portion thereof was supplied to a fifth column 500 in a gas purifying section including fifth and sixth columns.

Each of 10.395 wt% of isopropyl alcohol, 88.9 wt% of water, 0.5 wt% of diisopropyl ether (DIPE) as a first light by-product, 0.005 wt% of acetone as a second light by-product, and 0.2 wt% of n-propyl alcohol (NPA) and hexanol as heavy by-products was included in a feed stream 30 supplied to the first column 100.

Isopropyl alcohol was obtained by sequentially passing through a lower discharge stream of the first column 100, a first side discharge stream of the second column 200, a first region lower discharge stream of the third column 300, and an upper discharge stream of the fourth column 400. The diisopropyl ether (DIPE) was removed from an upper portion of the first column 100, and the acetone was removed from an upper portion of the second column 200. Meanwhile, water was removed from each of a lower discharge stream of the second column 200 and a lower portion of a second region of the third column 300. The heavy by-products were removed from each of a second side discharge stream of the second column 200 and a lower discharge stream of the fourth column 400.

Meanwhile, propylene and gas components were included in a stream 40 supplied to the fifth column 500. The propylene was recovered by sequentially passing through an upper discharge stream of the fifth column 500 and a side discharge stream of the sixth column 600.

Here, an upper discharge stream of the second column 200 was heat-exchanged with a lower discharge stream of the sixth column 600 in a first reboiler 610 of the sixth column. The heat-exchanged upper discharge stream of the second column was passed through a condenser 280 provided above the second column 200 and then branched into a stream refluxed to the second column 200 and a stream discharged outside the system.

At this time, the heat energy required for the operation of the sixth column was completely replaced by the heat energy supplied from the upper discharge stream of the second column, and thus, there was no need to supply a separate heat source to an auxiliary reboiler 615 for the operation of the sixth column.

Meanwhile, an upper discharge stream 330 of the third column 300 was branched into streams at a mass flow rate ratio of 1:6, and the branched streams were supplied to a first reboiler 110 of the first column and a first reboiler 210 of the second column, respectively, to supply heat energy to the lower portions of the first and second columns. At this time, an operating pressure of an upper region of the third column 300 was 5.0 bar.g, and a temperature of the upper discharge stream 330 of the third column 300 was 122°C. Meanwhile, a temperature of the lower discharge stream of the first column was 85°C, and a temperature of the lower discharge stream of the second column was 95°C. A temperature difference between a low-temperature medium and a high-temperature medium supplied to the first reboiler 110 of the first column and the first reboiler 210 of the second column was appropriate, and thus, efficient heat exchange was performed.

At this time, since the operating conditions of the first and second columns did not reach a desired level with only the heat energy of the upper discharge stream 330 of the third column, additional heat energy was supplied to the first column and the second column through auxiliary reboilers 115 and 215 provided below the first column and the second column, respectively.

Meanwhile, the first region lower discharge stream of the third column 300 including isopropyl alcohol and n-propyl alcohol was supplied to the fourth column 400, isopropyl alcohol was obtained from the upper portion of the fourth column 400, and a content of isopropyl alcohol in the upper discharge stream of the fourth column was confirmed to be 99.8 wt%.

The reboiler duties of the respective columns in this case were shown in Table 1. Specifically, since the heat energy supplied to the first reboiler 110 of the first column and the first reboiler 210 of the second column is supplied from the upper portion of the third column, as the reboiler duty in each of the first column and the second column, the heat energy supplied through the auxiliary reboilers 115 and 215 provided below the first column and the second column, respectively, is shown in Table 1. Meanwhile, the heat energy supplied through a reboiler 310 connected to the lower portion of the first region of the third column and the heat energy supplied through a reboiler 315 connected to the lower portion of the second region of the third column are shown in Table 1.

### Comparative Example 1

A process of preparing isopropyl alcohol was performed according to a process diagram of FIG. 5. A flow rate and composition of a stream introduced into a C1 column of an isopropyl alcohol purifying section were the same as the flow rate and composition of the stream introduced into the first column of Example 1, and a flow rate and composition of a stream introduced into a C7 column of a gas purifying section were the same as the flow rate and composition of the stream introduced into the fifth column of Example 1. Isopropyl alcohol of Comparative Example 1 was obtained from an upper portion of a C6 column, and the heat energy required for the operation of each column was measured to obtain 99.8 wt% of a content of isopropyl alcohol in an upper discharge stream of the C6 column as in Example 1, and the results were shown in Table 1.

Specifically, a lower discharge stream of the C1 column having the same composition as that of Example 1 was obtained and introduced into a C2 column.

The C2 column of Comparative Example 1 was a column not provided with a dividing wall, and as in Example 1, the stream was distilled to separate four discharge streams: an upper discharge stream of the C2 column including acetone, a first side discharge stream of the C2 column including an azeotrope of isopropyl alcohol and water, a second side discharge stream of the C2 column including n-propyl alcohol (NPA) and hexanol, and a lower discharge stream of the C2 column including water.

In this case, as a large amount of isopropyl alcohol was included in the second side discharge stream of the C2 column and was discharged without effectively separating the heavy by-products and water, a C3 column was required to additionally purify the isopropyl alcohol in the second side discharge stream of the C2 column and separate the heavy by-products and water. Specifically, the second side discharge stream of the C2 column was introduced into the C3 column, and a stream including isopropyl alcohol was separated from an upper portion of the C3 column and supplied again to the C2 column.

The energy required for the operation of the C1 to C3 columns was supplied through reboilers provided below the C1 to C3 columns.

Isopropyl alcohol in the first side discharge stream of the C2 column including an azeotrope of isopropyl alcohol and water was recovered from the upper portion of the C6 column using a general C4 column (azeotropic distillation column), C5 column (azeotropic agent recovery column), and C6 column (isopropyl alcohol recovery column) without a dividing wall. Specifically, isopropyl alcohol was obtained by sequentially passing through a lower discharge stream of the C4 column and an upper discharge stream of the C6 column. Each of the C4 to C6 columns was provided with a condenser above and a reboiler below.

Meanwhile, propylene and gas components were included in a stream supplied to the C7 column in the gas purifying section of Comparative Example 1. The propylene was recovered by sequentially passing through an upper discharge stream of the C7 column and a side discharge stream of a C8 column.

As a result, a content of isopropyl alcohol obtained in Comparative Example 1 was confirmed to be 99.8 wt% as in Example 1.

The energy (heat energy) used in the reboiler (reboiler duty) provided below each of the columns (C1 to C8 columns) in Comparative Example 1 in this case was shown in Table 1.

**[Table 1]**

| | Column | Reboiler duty (kW) | Total reboiler duty (kW) | Energy saving (%) |
|---|---|---|---|---|
| Comparative Example 1 | C1 | 8.9 | 100 | 0 |
| | C2 | 26.3 | | |
| | C3 | 4.5 | | |
| | C4 | 26.6 | | |
| | C5 | 13.4 | | |
| | C6 | 10.5 | | |
| | C7 | 3 | | |
| | C8 | 6.8 | | |
| Example 1 | First column | 2.3 | 29.5 | 70.6 |
| | Second column | 5.1 | | |
| | Third column (first region) | 11.5 | | |
| | Third column (second region) | 10.1 | | |
| | Fourth column | 0 | | |
| | Fifth column | 0.5 | | |
| | Sixth column | 0 | | |
| 1) The total energy consumption in Example 1 represents a relative energy consumption when the total energy consumption in Comparative Example 1 is set as 100. | | | | |
| 2) The amount of energy used in each reboiler of each column in Example 1 is a value obtained by distributing the total amount of energy used in Example 1 in proportion to the amount of energy actually used in each reboiler. | | | | |

As can be seen from the results above, in the case of Example 1, the purity and yield of isopropyl alcohol were maintained at a high level. In particular, in the case in which the heat of the upper discharge stream of the third column is used as an energy source required for the operation of the first and second columns while using dividing wall distillation columns having a specific structure as the second and third columns, it may be appreciated that the energy efficiency of the overall process from the first to fourth columns is maximized.

Furthermore, all or a portion of the heat energy required for the operation of the fifth column and the fourth column is replaced with the heat energy of the reaction product before being supplied to the absorption column, and all or a portion of the heat energy required for the operation of the sixth column is replaced with the heat energy of the upper discharge stream of the second column, such that the energy required for the entire process including reaction, purification, and recovery of unreacted substances for the preparation of isopropyl alcohol may be efficiently utilized.

### [Detailed Description of Main Elements]

| | | | |
|---|---|---|---|
| 100: | First column | 200: | Second column |
| 300: | Third column | 300: | Third column |
| 400: | Fourth column | 500: | Fifth column |
| 600: | Sixth column | | |

## Claims

1. A method for preparing isopropyl alcohol, the method comprising:
reacting a propylene monomer and water to prepare a reaction product including propylene and isopropyl alcohol;
cooling the reaction product and supplying the cooled reaction product to an absorption column; and
supplying a lower discharge stream of the absorption column including isopropyl alcohol from the absorption column to an isopropyl alcohol purifying section including first to fourth columns, and supplying an upper discharge stream of the absorption column including propylene to a gas purifying section including fifth and sixth columns,
wherein the isopropyl alcohol included in the lower discharge stream of the absorption column supplied to the isopropyl alcohol purifying section is obtained by sequentially passing through a lower discharge stream of the first column, a first side discharge stream of the second column, a first region lower discharge stream of the third column, and an upper discharge stream of the fourth column,
the propylene included in the upper discharge stream of the absorption column supplied to the gas purifying section is obtained by sequentially passing through an upper discharge stream of the fifth column and a side discharge stream of the sixth column,
all or a portion of the reaction product is cooled by heat exchange with one or more of a lower discharge stream of the fourth column and a side discharge stream of the fifth column,
an upper discharge stream of the second column is heat-exchanged with a lower discharge stream of the sixth column, and
an upper discharge stream of the third column is heat-exchanged with one or more of the lower discharge stream of the first column and a lower discharge stream of the second column.

2. The method of claim 1, wherein the lower discharge stream of the absorption column includes isopropyl alcohol, water, a first light by-product, a second light by-product, and a heavy by-product,
the first light by-product is separated from an upper discharge stream of the first column, and
the lower discharge stream of the first column including the isopropyl alcohol, the water, the second light by-product, and the heavy by-product is supplied to the second column.

3. The method of claim 2, wherein the first light by-product includes diisopropyl ether (DIPE), and
the second light by-product includes acetone.

4. The method of claim 1, wherein the second column includes a dividing wall spaced apart from the bottom and provided in a longitudinal direction of the column,
the second column is partitioned into a top region, a bottom region, a supply region, and a discharge region by the dividing wall,
the first side discharge stream and a second side discharge stream of the second column are discharged from the discharge region, and
the first side discharge stream is discharged from the discharge region at a higher position than the second side discharge stream.

5. The method of claim 4, wherein the first side discharge stream of the second column includes a mixture of isopropyl alcohol and water,
the second side discharge stream of the second column includes a heavy by-product, and
the heavy by-product includes n-propyl alcohol (NPA) and hexanol.

6. The method of claim 1, wherein the lower discharge stream of the second column includes water, and
a branched stream branched from a portion of the lower discharge stream of the second column is recycled to an upper portion of the first column and an upper portion of the absorption column.

7. The method of claim 6, wherein the lower discharge stream of the absorption column is supplied to the first column, and
a mass flow rate of a stream branched from a portion of the lower discharge stream of the second column and recycled to the upper portion of the first column is 0.4 to 1.2 with respect to a mass flow rate of the lower discharge stream of the absorption column supplied to the first column.

8. The method of claim 1, wherein the third column includes a dividing wall connected to the bottom and extending in a longitudinal direction of the column, and
the third column is partitioned, by the dividing wall, into a first region, a second region facing the first region, and an upper region located above an upper end of the dividing wall.

9. The method of claim 1, wherein the upper discharge stream of the third column is heat-exchanged with the lower discharge stream of the first column and the lower discharge stream of the second column through a first reboiler of the first column and a first reboiler of the second column.

10. The method of claim **9,** wherein the upper discharge stream of the third column is branched to form a branched stream supplied to the first reboiler of the first column and a branched stream supplied to the first reboiler of the second column, and
a ratio of a mass flow rate of the branched stream supplied to the first reboiler and a mass flow rate of the branched stream supplied to the second reboiler is 1:5 to 1:7.

11. The method of claim 1, wherein the first region lower discharge stream of the third column is supplied to the fourth column,
isopropyl alcohol is obtained through an upper portion of the fourth column, and
a heavy by-product is separated through a lower portion of the fourth column.

12. The method of claim 1, wherein the cooling of the reaction product is performed by including a first cooling and a second cooling, the first cooling being performed by heat exchange between all or a portion of a reaction product stream and one or more of the lower discharge stream of the fourth column and the side discharge stream of the fifth column, and the second cooling being performed by heat exchange between the reaction product stream that is subjected to the first cooling and a refrigerant.

13. The method of claim 12, wherein the fifth column includes a first reboiler of the fifth column provided beside the fifth column and a second reboiler of the fifth column provided below the fifth column, and
the heat exchange between all or a portion of the reaction product stream and the side discharge stream of the fifth column is performed in the first reboiler of the fifth column.

14. The method of claim 12, wherein the reaction product stream is branched into a first branched stream, a second branched stream, and a third branched stream,
the first cooling is performed by heat exchange between the first branched stream and the lower discharge stream of the fourth column and heat exchange between the second branched stream and the side discharge stream of the fifth column,
the first and second branched streams that are subjected to the first cooling are combined with the third branched stream that is not subjected to the first cooling to form a combined stream, and
the second cooling is performed by heat exchange between the combined stream and the refrigerant.

15. The method of claim 14, wherein a mass flow rate of the first branched stream:a mass flow rate of the second branched stream:a mass flow rate of the third branched stream is 1:0.2 to 0.4:0.4 to 0.7.
